# EUROPÄISCHE PATENTSCHRIFT

(11) **EP 0 456 603 B1**
(45) Veröffentlichungstag und Bekanntmachung des Hinweises auf die Patenterteilung: **21.06.1995**
(21) Anmeldenummer: 91810314.4
(22) Anmeldetag: 25.04.1991
(51) Int. Cl.: C07D 303/36, C08G 59/50

(54) **Tetra-N-glylcidylverbindung und deren härtbaren Gemische**
Tetra-N-glycedyl compound and its hardenable mixtures
Composés tétra-N-glycidyliques et leurs mélanges durcissables

(30) Priorität: 05.05.1990 GB 9010221
(43) Veröffentlichungstag der Anmeldung: 13.11.1991
(73) Patentinhaber: CIBA-GEIGY AG, 4002 Basel (CH)
(72) Erfinder: Dobinson, Bryan, Dr., Duxford, Cambridge CB2 4PX (GB); Thoseby, Michael Robert, Dr., Cambridge CB4 1HW (GB)

(56) Entgegenhaltungen:
- EP-A- 0 148 117
- GB-A- 874 750
- GB-A- 2 111 977

## Beschreibung

Die vorliegende Erfindung betrifft die Tetra-N-glycidylverbindung des 3,3'-Diethyl-4,4'-diaminodiphenylmethans und diese Verbindung enthaltende härtbare Epoxidharzgemische.

Epoxidharze haben in der Industrie vielfältige Anwendung gefunden, beispielsweise als Klebstoffe, Beschichtungen, Giessharze, Isolationsharze und in verstärkten Verbundstoffen. Für diese Anwendungen gibt es eine Vielzahl chemisch verschiedener Epoxidharze. Im allgemeinen werden Ether oder Ester aus Epichlorhydrin und einem Bisphenol A oder einer Dicarbonsäure eingesetzt. Wird ein gutes Verhalten bei hoher Temperatur vorausgesetzt, wie beispielsweise in der Flugzeugindustrie, wird oft ein Epoxidharz bevorzugt, dessen Glycidylgruppen an aromatische Aminogruppen substituiert sind. Solche Epoxidharze werden bekanntlich durch Umsetzung eines aromatischen Amins mit Epichlorhydrin erhalten, wobei pro 1 Äquivalent Aminwasserstoffatom 0,8-10 Äquivalente Epichlorhydrin eingesetzt werden und anschliessend in bekannter Weise die Dehydrochlorierung mittels Alkali vorgenommen wird. Die Umsetzung kann in Gegenwart eines sauren Katalysators vorgenommen werden, wie sie beispielsweise im GB-Patent 2,111,977 beschrieben wird.

Im EP-Patent 143,075 wird ein Verfahren zur Herstellung von N-Glycidylverbindungen mit einem vergleichsweise höheren Epoxidgehalt und einer tieferen Viskosität beschrieben. Dieses Verfauren wird durchgeführt, indem die Umsetzung des aromatischen Amins mit wenigstens 0,7 Äquivalenten, vorzugsweise 0,8 bis 1,5 Äquivalenten Epichlorhydrin pro Aminwasserstoffäquivalent des aromatischen Amins in Gegenwart a) eines di- oder höherwertigen Metallsalzes der Salpeter- oder Perchlorsäure oder b) einer Carbon- oder Sulfonsäure, deren α-Kohlenstoffatom zur Säuregruppe durch Fluor, Chlor oder Brom substituiert ist.

Es wurde nun gefunden, dass durch Umsetzung von 3,3'-Diethyl-4,4'-diaminodiphenylmethan mit Epichlorhydrin N,N,N'N'-Tetraglycidyl-3,3'-diethyl-4,4'-diaminodiphenylmethan als neue Verbindung erhalten wird, die bessere Eigenschaften, insbesondere eine bessere Viskosität, im Vergleich zu den N-Glycidylverbindungen aus dem EP-Patent 143,075 aufweist.

Gegenstand vorliegender Erfindung ist somit die Verbindung N,N,N'N'-Tetraglycidyl-3,3'-diethyl-4,4'-diaminodiphenylmethan, welche der Formel I entspricht.

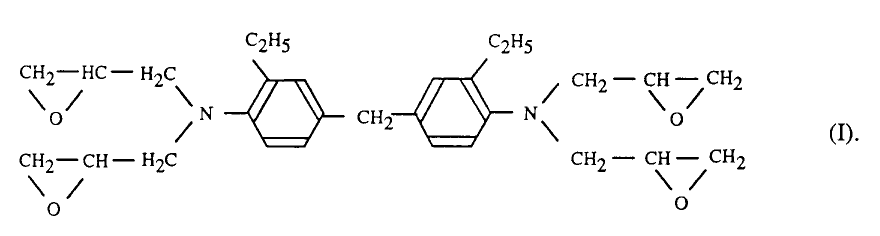

Während in der Literatur allgemein angegebene Formeln die spezifische Verbindung der Formel I mitumfassen und auch Reaktionsgemische, enthaltend Anteile an der Verbindung der Formel I beispielsweise aus der EP-A-0 143 075 und der JP-A-074 552 bekannt sind, ist die reine Verbindung der Formel I noch nicht hergestellt und beschrieben worden.

Die Verbindung der Formel I kann durch Umsetzung von 3,3'-Diethyl-4,4'-diaminodiphenylmethan mit Epichlorhydrin, vorzugsweise in Gegenwart eines Katalysators, insbesondere eines in der EP-A-143 075 beschriebenen Katalysators, hergestellt werden.

Bevorzugte Salze, die für die Herstellung der Verbindung der Formel I verwendet werden, sind Magnesiumperchlorat, Calciumperchlorat, Zinkperchlorat, Nickelperchlorat, Magnesiumnitrat, Mangannitrat, Lanthannitrat, Ytterbiumnitrat, Urannitrat, Magnesiumtrifluoracetat, Mangantrifluoracetat, Nickeltrifluoracetat, Vanadiumtrifluoracetat, Magnesiumtrifluormethansulphonat, Kolbalttrifluormethansulphonat, Lanthantrifluoracetat, Lanthantrifluormethansulphonat, Magnesiumtrichloracetat, Magnesium-2,2-dichlorpropionat und Magnesiumtribromacetat.

Die bei der Umsetzung von 3,3'-Diethyl-4,4'-diaminodiphenylmethan mit Epichlorhydrin verwendete Menge eines oben genannten Salzes beträgt im allgemeinen 0,1 bis 10 Teile, vorzugsweise 0,4 bis 2 Teile, pro 100 Teile des 3,3'-Diethyl-4,4'-diaminodiphenylmethans.

Vorzugsweise werden die Katalysatoren dem Reaktionsgemisch in einem inerten Lösungsmittel gelöst zugegeben. Beispiele für inerte Lösungsmittel sind 2-Methoxyethanol, Isodecanol, Ethylenglykol, Diethylenglykol, N-Methylpyrrolidon, γ-Butyrolacton, Benzylalkohol, Dibutylphthalat, Butan-1,4-diol, Ethylmethylketon, Benzol und Toluol.

Die Umsetzung wird im allgemeinen bei erhöhter Temperatur durchgeführt, vorzugsweise im Bereich von 50 bis 100°C. Wenn die Reaktion zwischen 3,3'-Diethyl-4,4'-diaminodiphenylmethan und Epichlorhydrin vollständig ist, im allgemeinen zwischen 1 bis 12 Stunden, wird die Dehydrochlorierung in bekannter Weise durchgeführt, beispielsweise druch Zugabe von Natrium- oder Kaliumhydroxid, gegebenenfalls in Gegenwart eines quaternären Ammoniumhalogenids als Phasentransferkatalysator, wie beispielsweise Benzyltrimethylammoniumchloride. Nach dem Erhitzen, beispielsweise während 2 bis 10 Stunden auf 50 bis 100°C, wird das Reaktionsgemisch im allgemeinen mit Wasser gewaschen und die organische, die Tetraglycidylverbindung enthaltende Phase abgetrennt. Aus dieser wird die Verbindung der Formel I in bekannter Weise isoliert und gereinigt.

Die vorliegende Erfindung betrifft auch härtbare Epoxidharzgemische, enthaltend
(a) N,N,N'N'-Tetraglycidyl-3,3'-diethyl-4,4'-diaminodiphenylmethan,
(b) ein Härtungsmittel für Epoxidharze und gegebenenfalls
(c) einen Härtungsbeschleuniger.

Das Härtungsmittel ist vorzugsweise eine stickstoffhaltige Verbindung, die gegenüber der Verbindung der Formel I bis zu einer gewissen Schwellentemperatur, im allgemeinen bis wenigstens 80°C, vorzugsweise wenigstens bis 100°C, inert ist, doch oberhalb der Schwellentemperatur schnell mit der Epoxidverbindung reagiert. Solche Verbindungen sind bekannt und zum Teil im Handel erhältlich. Beispielsweise eignen sich dafür Bortrichlorid/Amin- und Bortrifluorid/Amin-Komplexe; Dicyandiamid; Melamin; Diallylmelamin; Guanamine, wie Acetoguanamin und Benzoguanamin; Aminotriazole, wie 3-Amino-1,2,4-triazol; Hydrazide, wie Adipin-, Stearin- und Isophthalisäurehydrazide; Semicarbazid; Cyanoacetamid; und aromatische Polyamine, wie Diaminodiphenylsulphon. Das Härtungsmittel b) kann auch saurer Natur sein, wie beispielsweise Polyphenole, Polycarbonsäuren und vorzugsweise Di- und Polycarbonsäureanhydride, insbesondere Hexahydrophthalsäureanhydrid oder Methyltetrahydrophthalsäureanhydrid.

Das härtbare, erfindungsgemässe Gemisch enthält im allgemeinen 1 bis 60 Gew.-%, vorzugsweise 5 bis 50 Gew.-%, des Härtungsmittels (b), bezogen auf die Menge der Epoxidharzkomponente (a).

Die gegebenenfalls in den erfindungsgemässen Gemischen enthaltenen Härtungsbeschleuniger sind bekannt und deren Wahl hängt vom Typ und der Reaktivität der Komponente (b) ab. Beispielsweise eignen sich als Härtungsbeschleuniger feste Lösungen aus einer Stickstoffbase mit einem Siedepunkt über 130°C und einem phenolischen Polymer, das ein Polymerisationsprodukt aus einem Phenol mit ungesättigtem Substituenten ist und im EP-Patent 200 678 beschrieben wird, sowie Reaktionsprodukte aus einer Stickstoffbase und einem monomeren Phenol mit Halogensubstituenten, wie sie im EP-Patent 240 459 beschrieben werden. Weitere Beispiele für Härtungsbeschleuniger sind latente Lewissäure-Komplexe, wie beispielsweise BF₃-Aminkomplex, latente Basen, wie substituierter Harnstoff, beispielsweise Monuron oder Chlortoluron, ausserdem N,N-Dimethylharnstoff, und BCl₃-tert.-Aminkomplexe, ferner basische Beschleuniger, wie tertiäre Amine.

Vorzugsweise enthalten die erfindungsgemässen Gemische als Härtungsbeschleuniger Monuron, Chlortoluron, ein BF₃-Aminkomplex, ein BCl₃-tert.-Aminkomplex oder ein tert.Amin.

Die Menge des Härtungsmittels c) ist nicht kritisch und kann, bezogen auf die Gewichtsmenge der Tetraglycidylverbindung a), zwischen 0 bis 15 Gew.-%, vorzugsweise zwischen 0 bis 10 Gew.-%, betragen.

Die erfindungsgemässen Gemische können auch ein oder mehrere geeignete Plastifizierungsmittel, wie Dibutyl- oder Dioctylphthalat, Verdünnungsmittel, wie Teer oder Bitumen, oder sogenannte reaktive Verdünner insbesondere Monoepoxide, wie beispielsweise n-Butylglycidylether, iso-Octylglycidylether, Phenylglycidylether, Kresylglycidylether, Glycidylester von gemischten tertiären aliphatischen Monocarbonsäuren, Glycidylacrylate oder -methacrylate enthalten. Die erfindungsgemässen Gemische können auch andere Polyglycidylverbindungen, wie Glycidylderivate von merhwertigen Phenolen, Polyolen oder Polycarbonsäuren, enthalten. Die erfindungsgemässen Gemische können auch Additive, wie Füllstoffe, Verstärkungsmittel, polymere Zähigkeitsmittel, wie Polyethersulfone, Phenoxyharze und Butadien-Acrylnitril-Kautschuk, Farbstoffe, Fliessmittel, Flammschutzmittel und Formtrennmittel, enthalten.

Geeignete Streckungsmittel, Füllstoffe und Verstärkungsmittel sind beispielsweise Glasfasern, Kohlenstoffasern, aromatische Polyamidfasern, Ballotini®, Glimmer, Quartz, Calciumcarbonat, Zellulose, Kaolin, Wollastonit, kolloidale Kieselerde mit einer grossen spezifischen Oberfläche, gepulvertes Polyvinylchlorid und gepulverte Polyolefine, wie Polyethylen oder Polypropylen.

Die erfindungsgemässen Gemische eignen sich als Laminierharze, Imprägnierharze, Giessharze, Formmassen, für Pulverbeschichtungen, als Kitt oder Spachtelmassen oder als Isolationsmaterial für die elektrische Industrie, insbesondere als Giess-, Laminier- oder Imprägnierharze.

Die erfindungsgemässen Gemische können in bekannter Weise durch Erhitzen im Temperaturbereich von 100 bis 200°C, vorzugsweise von 150 bis 180°C, gehärtet werden. Normalerweise genügt dabei ein Erhitzen von 60 bis 180 Minuten zum Aushärten, doch kann ein Nachhärten bei höheren Temperaturen zur Erzielung von optimalen Eigenschaften erforderlich sein.

Im Vergleich zu den bekannten Epoxidharzen weist die erfindungsgemässe Verbindung eine geringere Viskosität mit verbesserten Verarbeitungseigenschaften und eine wesentliche Verbesserung der Lagerstabilität im Gemisch mit 4,4'-Diaminodiphenylsulfon auf, ohne dass sich der Tg-Wert im wesentlichen verschlechtert.

Die folgenden Beispiele erläutern die Erfindung etwas mehr.

### Beispiel 1

100 g 3,3'-Diethyl-4,4'-diaminodiphenylmethan, 150 g Toluol und 2 g einer 50 gew.-%igen Lösung von Lanthannitrat in 2-Methoxyethanol werden gerührt und unter Vakuum von 156 mbar auf 60°C erhitzt. Dann werden 158,6 g Epichlorhydrin während einer Stunde hinzugegeben, wobei die Temperatur auf 60°C unter Beibehaltung des Vakuums gehalten wird. Nach Beendigung der Zugabe wird das Reaktionsgemisch während 15 Minuten auf 60°C gehalten und danach das Vakuum aufgehoben. Dann werden weitere 2 g des Katalysators zugegeben und die Temperatur auf 80°C gehoben und während 3 Stunden auf 80°C gehalten. Dann wird die Temperatur auf 60°C erniedrigt und 1,5 g einer 50%igen wässrigen Benzyltrimethylammoniumchloridlösung werden zugegeben und die Apparatur für die Vakuumdestillation eingestellt. Es werden 151 g einer 50%igen wässrigen Natronlauge während 3 Stunden zugegeben, und das Wasser wird unter Vakuum (133 mbar) azeotrop entfernt. Nach der Zugabe wird die azeotrope Destillation nach 90 Minuten fortgesetzt. Unter kräftigem Rühren werden dann 300 ml Wasser zugesetzt und die wässrige Schicht wird dann abdekantiert. Die organische Phase wird mit 200 ml einer 10%igen wässrigen Natriumdihydrogenphosphatlösung gewaschen und am Rotationsverdampfer von Wasserspuren befreit. Der Rückstand wird in 250 ml Toluol gelöst, filtriert und unter Vakuum eingeengt, wobei ein Epoxidharz mit einem Epoxidgehalt von 7,89 Mol/kg (94,9 % der Theorie) mit einer Viskosität von 7,1 Pa·s bei 25°C und einem Monomergehalt von 92,9 %, bestimmt durch HPLC-Messung, erhalten wird.

### Beispiel 2

10 des gemäss Beispiel 1 hergestellten Epoxidharzes, 4,9 g 4,4'-Diaminodiphenylsulfon und 0,3 g Aerosil® R 805 werden zusammen auf einem Dreiwalzenstuhl gemahlen. Eine Probe dieser Mischung wird bei 60°C aufbewahrt, wobei in periodischen Abständen geprüft wird, ob die Mischung eine für die Weiterarbeitung noch ausreichend niedrige Viskosität aufweist. Nach 73 Tagen weist die Mischung eine Viskosität auf, die für die Verarbeitung der Mischung noch geeignet ist. Eine weitere Probe wird während 3 Stunden bei 175°C und 2 Stunden bei 205°C gehärtet. Die gehärtete Probe weist einen Tg-Wert von 261°C auf.

Eine analoge Mischung, welche die Tetraglycidylverbindung des 4,4'-Diaminodiphenylmethans, welche eine Viskosität von 100,7 Pa·s bei 25°C aufweist, enthält, hat bei 60°C eine Lagerstabilität von 10 Tagen, und nach Aushärtung unter gleichen Bedingungen weist die gehärtete Probe einen Tg-Wert von 260°C auf.

## Patentansprüche (Patentansprüche für folgende(n) Vertragsstaat(en): DE, FR, GB, IT, NL, SE)

1. N,N,N'N'-Tetraglycidyl-3,3'-diethyl-4,4'-diaminodiphenylmethan.

2. Härtbares Gemisch, enthaltend
(a) N,N,N'N'-Tetraglycidyl-3,3'-diethyl-4,4'-diaminodiphenylmethan,
(b) ein Härtungsmittel für Epoxidharze und gegebenenfalls
(c) einen Härtungsbeschleuniger.

3. Gemisch gemäss Anspruch 2, worin das Härtungsmaterial (b) eine stickstoffhaltige Verbindung ist, die gegenüber N,N,N'N'-Tetraglycidyl-3,3'-diethyl-4,4'-diaminodiphenylmethan unterhalb einer Temperatur von mindestens 80°C inert ist.

4. Gemisch gemäss Anspruch 3, worin das Härtungsmittel (b) ein Bortrichlorid/Amin- oder Bortrifluor/Amin-Komplex, Dicyandiamin, Melamin, Dialkylmelamin, ein Guanamin, ein Aminotriazol, ein Hydrazid, Semicarbazid, Cyanoacetamid oder ein aromatisches Polyamin ist.

5. Gemisch gemäss Anspruch 4, das als Guanamin Acetoguanamin oder Benzoguanamin, als Aminotriazol 3-Amino-1,2,4-triazol, als Hydrazid Adipin-, Stearin- oder Isophthalsäurehydrazid und als aromatisches Polyamin Diaminodiphenylsulfon enthält.

6. Gemisch gemäss Anspruch 2, enthaltend als Härtungsmittel (b) eine Polycarbonsäure oder ein Polycarbonsäureanhydrid.

7. Gemisch gemäss Anspruch 6, enthaltend als Polycarbonsäureanhydrid Hexahydrophthalsäure- oder Tetrahydrophthalsäureanhydrid.

8. Gemisch gemäss Anspruch 2, enthaltend 1 bis 60 Gew.-% Härtungsmittel (b), bezogen auf die Menge der Komponente (a).

9. Gemisch gemäss Anspruch 2, enthaltend als Härtungsbeschleuniger eine feste Lösung aus einer Stickstoffbase mit einem Siedepunkt von 130°C und einem phenolischen Polymer, das ein Polymerisationsprodukt eines Phenols mit einem ungesättigten Substituenten ist, ein Reaktionsprodukt aus einer Stickstoffbase und einem halogensubstituierten Phenol, einen latenten Lewissäure-Komplex, eine latente Base oder einen basischen Beschleuniger.

10. Gemsich gemäss Anspruch 9, enthaltend Monuron oder Chlortoluron als substituierten Harnstoff, einen BF₃-Aminkomplex, einen BCl₃-tert.-Aminokomplex oder ein tert.-Amin.

11. Gemisch gemäss Anspruch 2, enthaltend 0 bis 15 Gew.-% eines Härtungsbeschleunigers (c), bezogen auf die Komponente a).

12. Verwendung des Gemisches gemäss Anspruch 2 als Laminier-, Imprägnier- oder Giessharz, zur Pulverbeschichtung, als Formmasse, als Spachtel- oder Dichtungsmasse und als Isoliermaterial für die Elektroindustrie.

## Patentansprüche (Patentansprüche für folgende(n) Vertragsstaat(en): ES)

1. Härtbares Gemisch, enthaltend
(a) N,N,N'N'-Tetraglycidyl-3,3'-diethyl-4,4'-diaminodiphenylmethan,
(b) ein Härtungsmittel für Epoxidharze und gegebenenfalls
(c) einen Härtungsbeschleuniger.

2. Gemisch gemäss Anspruch 1, worin das Härtungsmaterial (b) eine stickstoffhaltige Verbindung ist, die gegenüber N,N,N'N'-Tetraglycidyl-3,3'-diethyl-4,4'-diaminodiphenylmethan unterhalb einer Temperatur von mindestens 80°C inert ist.

3. Gemisch gemäss Anspruch 2, worin das Härtungsmittel (b) ein Bortrichlorid/Amin- oder Bortrifluor/Amin-Komplex, Dicyandiamin, Melamin, Dialkylmelamin, ein Guanamin, ein Aminotriazol, ein Hydrazid, Semicarbazid, Cyanoacetamid oder ein aromatisches Polyamin ist.

4. Gemisch gemäss Anspruch 3, das als Guanamin Acetoguanamin oder Benzoguanamin, als Aminotriazol 3-Amino-1,2,4-triazol, als Hydrazid Adipin-, Stearin- oder Isophthalsäurehydrazid und als aromatisches Polyamin Diaminodiphenylsulfon enthält.

5. Gemisch gemäss Anspruch 1, enthaltend als Härtungsmittel (b) eine Polycarbonsäure oder ein Polycarbonsäureanhydrid.

6. Gemisch gemäss Anspruch 5, enthaltend als Polycarbonsäureanhydrid Hexahydrophthalsäure- oder Tetrahydrophthalsäureanhydrid.

7. Gemisch gemäss Anspruch 1, enthaltend 1 bis 60 Gew.-% Härtungsmittel (b), bezogen auf die Menge der Komponente (a).

8. Gemisch gemäss Anspruch 1, enthaltend als Härtungsbeschleuniger eine feste Lösung aus einer Stickstoffbase mit einem Siedepunkt von 130°C und einem phenolischen Polymer, das ein Polymerisationsprodukt eines Phenols mit einem ungesättigten Substituenten ist, ein Reaktionsprodukt aus einer Stickstoffbase und einem halogensubstituierten Phenol, einen latenten Lewissäure-Komplex, eine latente Base oder einen basischen Beschleuniger.

9. Gemsich gemäss Anspruch 8, enthaltend Monuron oder Chlortoluron als substituierten Harnstoff, einen BF₃-Aminkomplex, einen BCl₃-tert.-Aminokomplex oder ein tert.-Amin.

10. Gemisch gemäss Anspruch 1, enthaltend 0 bis 15 Gew.-% eines Härtungsbeschleunigers (c), bezogen auf die Komponente a).

## Claims (Claims for the following Contracting State(s): DE, FR, GB, IT, NL, SE)

1. N,N,N',N'-tetraglycidyl-3,3'-diethyl-4,4'-diaminodiphenylmethane.

2. Curable mixture comprising
(a) N,N,N',N'-tetraglycidyl-3,3'-diethyl-4,4'-diaminodiphenylmethane,
(b) a curing agent for epoxy resins, and, if desired,
(c) a curing accelerator.

3. Mixture according to claim 2, in which the curing agent (b) is a nitrogen-containing compound which is inert towards N,N,N',N'-tetraglycidyl-3,3'-diethyl-4,4'-diaminodiphenylmethane below a temperature of at least 80°C.

4. Mixture according to claim 3, in which the curing agent (b) is a boron trichloride/amine or boron trifluoride/amine complex, dicyandiamine, melamine, dialkylmelamine, a guanamine, an aminotriazole, a hydrazide, semicarbazide, cyanoacetamide or an aromatic polyamine.

5. Mixture according to claim 4, which contains acetoguanamine or benzoguanamine as guanamine, 3-amino-1,2,4-triazole as aminotriazole, adipohydrazide, stearohydrazide or isophthalohydrazide as hydrazide, and diaminodiphenyl sulfone as aromatic polyamine.

6. Mixture according to claim 2, wherein the curing agent (b) is a polycarboxylic acid or a polycarboxylic anhydride.

7. Mixture according to claim 6, wherein the polycarboxylic anhydride is hexahydrophthalic or tetrahydrophthalic anhydride.

8. Mixture according to claim 2, containing from 1 to 60% by weight of curing agent (b), based on the amount of component (a).

9. Mixture according to claim 2, wherein the curing accelerator is a solid solution of a nitrogen base having a boiling point of 130°C and a phenolic polymer which is a product of the polymerization of a phenol having an unsaturated substituent, a product of the reaction of a nitrogen base and a halogen-substituted phenol, a latent Lewis acid complex, a latent base or a basic accelerator.

10. Mixture according to claim 9, containing monuron or chlorotoluron as substituted urea, a BF₃/amine complex, a BCl₃/tert-amino complex or a tert-amine.

11. Mixture according to claim 2, containing from 0 to 15% by weight of a curing accelerator (c), based on component a).

12. Use of the mixture according to claim 2 as a laminating, impregnating or casting resin, for powder coating, as a moulding composition, as a knifing filler or sealant, and as an insulating material for the electrical industry.

## Claims (Claims for the following Contracting State(s): ES)

1. Curable mixture comprising
(a) N,N,N',N'-tetraglycidyl-3,3'-diethyl-4,4'-diaminodiphenylmethane,
(b) a curing agent for epoxy resins, and, if desired,
(c) a curing accelerator.

2. Mixture according to claim 1, in which the curing agent (b) is a nitrogen-containing compound which is inert towards N,N,N',N'-tetraglycidyl-3,3'-diethyl-4,4'-diaminodiphenylmethane below a temperature of at least 80°C.

3. Mixture according to claim 2, in which the curing agent (b) is a boron trichloride/amine or boron trifluoride/amine complex, dicyandiamine, melamine, dialkylmelamine, a guanamine, an aminotriazole, a hydrazide, semicarbazide, cyanoacetamide or an aromatic polyamine.

4. Mixture according to claim 3, which contains acetoguanamine or benzoguanamine as guanamine, 3-amino-1,2,4-triazole as aminotriazole, adipohydrazide, stearohydrazide or isophthalohydrazide as hydrazide, and diaminodiphenyl sulfone as aromatic polyamine.

5. Mixture according to claim 1, wherein the curing agent (b) is a polycarboxylic acid or a polycarboxylic anhydride.

6. Mixture according to claim 5, wherein the polycarboxylic anhydride is hexahydrophthalic or tetrahydrophthalic anhydride.

7. Mixture according to claim 1, containing from 1 to 60% by weight of curing agent (b), based on the amount of component (a).

8. Mixture according to claim 1, wherein the curing accelerator is a solid solution of a nitrogen base having a boiling point of 130°C and a phenolic polymer which is a product of the polymerization of a phenol having an unsaturated substituent, a product of the reaction of a nitrogen base and a halogen-substituted phenol, a latent Lewis acid complex, a latent base or a basic accelerator.

9. Mixture according to claim 8, containing monuron or chlorotoluron as substituted urea, a BF₃/amine complex, as BCl₃/tert-amino complex or a tert-amine.

10. Mixture according to claim 1, containing from 0 to 15% by weight of a curing accelerator (c), based on component a).

## Revendications (Revendications pour l'(les) Etat(s) contractant(s) suivant(s): DE, FR, GB, IT, NL, SE)

1. N,N,N'N'-tétraglycidyl-3,3'-diéthyl-4,4'-diaminodiphénylméthane.

2. Mélange durcissable, contenant
(a) le N,N,N'N'-tétraglycidyl-3,3'-diéthyl-4,4'-diaminodiphénylméthane,
(b) un agent de durcissement pour résine époxy et éventuellement
(c) un accélérateur de durcissement.

3. Mélange selon la revendication 2, dans lequel le matériau de durcissement (b) est un composé contenant de l'azote qui est inerte vis à vis du N,N,N'N'-tétraglycidyl-3,3'-diéthyl-4,4'-diaminodiphénylméthane au dessous d'une température d'au moins 80°C.

4. Mélange selon la revendication 3, dans lequel l'agent de durcissement (b) est un complexe trichlorure de bore/amine ou trifluorure de bore/amine, le dicyandiamine, la mélamine, des dialkyl-mélamines, une guanamine, un aminotriazole, un hydrazide, un semicarbazide, un cyanoacétamide ou une polyamine aromatique.

5. Mélange selon la revendication 4, qui contient, en tant que guanamine, l'acétoguanamine ou la benzoguanamine, en tant qu'aminotriazole, le 3-amino-1,2,4-triazole, en tant qu'hydrazide, l'hydrazide de l'acide adipique, de l'acide stéarique ou de l'acide isophtalique et, en tant que polyamine aromatique, la diaminodiphénylsulfone.

6. Mélange selon la revendication 2, contenant, en tant qu'agent de durcissement (b), un acide polycarboxylique ou un anhydride d'acide polycarboxylique.

7. Mélange selon la revendication 6, contenant, en tant qu'anhydride d'acide polycarboxylique, l'anhydride de l'acide hexahydrophtalique ou l'anhydride de l'acide tétrahydrophtalique.

8. Mélange selon la revendication 2, contenant de 1 à 60% en poids de l'agent de durcissement (b), par rapport à la quantité du composant (a).

9. Mélange selon la revendication 2, contenant, en tant qu'accélérateur de durcissement, une solution solide d'une base azotée ayant un point d'ébullition de 130°C et d'un polymère phénolique, qui est le produit de polymérisation d'un phénol ayant un substituant insaturé, un produit de réaction d'une base azotée et d'un phénol halogéno-substitué, d'un complexe d'acide de Lewis latent, d'une base latente ou d'un accélérateur basique.

10. Mélange selon la revendication 9, contenant du monuron ou du chlortoluron en tant qu'urée substituée, un complexe BF₃-amine, un complexe BCl₃-amino tertiaire ou une amine tertiaire.

11. Mélange selon la revendication 2, contenant de 0 à 15% en poids d'un accélérateur de durcissement (c), par rapport au composant a).

12. Utilisation du mélange selon la revendication 2, en tant que résine pour stratifié, de résine d'imprégnation ou de résine de coulée, pour des enductions en poudre, en tant que masse moulable, en tant que mastic ou matériau d'étanchéité et en tant que matériau d'isolation pour l'industrie électronique.

## Revendications (Revendications pour l'(les) Etat(s) contractant(s) suivant(s): ES)

1. Mélange durcissable, contenant
(a) le N,N,N'N'-tétraglycidyl-3,3'-diéthyl-4,4'-diaminodiphénylméthane,
(b) un agent de durcissement pour résine époxy et éventuellement
(c) un accélérateur de durcissement.

2. Mélange selon la revendication 1, dans lequel le matériau de durcissement (b) est un composé contenant de l'azote qui est inerte vis à vis du N,N,N'N'-tétraglycidyl-3,3'-diéthyl-4,4'-diaminodiphénylméthane au dessous d'une température d'au moins 80°C.

3. Mélange selon la revendication 2, dans lequel l'agent de durcissement (b) est un complexe trichlorure de bore/amine ou trifluorure de bore/amine, le dicyandiamine, la mélamine, des dialkyl-mélamines, une guanamine, un aminotriazole, un hydrazide, un semicarbazide, un cyanoacétamide ou une polyamine aromatique.

4. Mélange selon la revendication 3, qui contient, en tant que guanamine, l'acétoguanamine ou la benzoguanamine, en tant qu'aminotriazole, le 3-amino-1,2,4-triazole, en tant qu'hydrazide, l'hydrazide de l'acide adipique, de l'acide stéarique ou de l'acide isophtalique et, en tant que polyamine aromatique, la diaminodiphénylsulfone.

5. Mélange selon la revendication 1, contenant, en tant qu'agent de durcissement (b), un acide polycarboxylique ou un anhydride d'acide polycarboxylique.

6. Mélange selon la revendication 5, contenant, en tant qu'anhydride d'acide polycarboxylique, l'anhydride de l'acide hexahydrophtalique ou l'anhydride de l'acide tétrahydrophtalique.

7. Mélange selon la revendication 1, contenant, de 1 à 60% en poids de l'agent de durcissement (b), par rapport à la quantité du composant (a).

8. Mélange selon la revendication 1, contenant, en tant qu'accélérateur de durcissement, une solution solide d'une base azotée ayant un point d'ébullition de 130°C et d'un polymère phénolique, qui est le produit de polymérisation d'un phénol ayant un substituant insaturé, un produit de réaction d'une base azotée et d'un phénol halogéno-substitué, d'un complexe d'acide de Lewis latent, d'une base latente ou d'un accélérateur basique.

9. Mélange selon la revendication 8, contenant du monuron ou du chlortoluron en tant qu'urée substituée, un complexe BF₃-amine, un complexe BCl₃-amino tertiaire ou une amine tertiaire.

10. Mélange selon la revendication 1, contenant de 0 à 15% en poids d'un accélérateur de durcissement (c), par rapport au composant a).
